(19) Europäisches Patentamt
European Patent Office
Office européen des brevets

(11) Publication number: 0 052 086
B1

(12) # EUROPEAN PATENT SPECIFICATION

(45) Date of publication of patent specification: 29.01.86

(51) Int. Cl.[4]: C 07 H 17/06, C 07 D 311/32

(21) Application number: 81830217.6

(22) Date of filing: 05.11.81

(54) **Process for producing flavone derivatives with medicinal activity.**

(30) Priority: 11.11.80 IT 2586880

(43) Date of publication of application:
19.05.82 Bulletin 82/20

(45) Publication of the grant of the patent:
29.01.86 Bulletin 86/05

(84) Designated Contracting States:
AT BE CH DE FR GB LI LU NL SE

(56) References cited:
FR-A-2 255 894
FR-A-2 402 655

CHEMICAL ABSTRACTS, vol. 73, no. 3, July 20, 1970, page 344, ref. 14620w, Columbus, Ohio, US, G. WURM et al.: "Synthesis and properties of symmetric di(2-chromonyl)benzenes"

CHEMICAL ABSTRACTS, vol. 34, no. 13, July 10, 1940, ref. 4735 (5), Columbus, Ohio, US, DUHKHAHARAN CHAKRAVARTI et al.: "Pyrones. I. Attempted oxidation of chromanones with selenium dioxide"

(73) Proprietor: BONOMELLI S.p.A.
Viale Montecuccoli, 1
I-22042 Dolzago Como (IT)

(72) Inventor: Redaelli, Claudio
Via Vittorio Veneto 15
Perego (Como) (IT)

(74) Representative: Gervasi, Gemma et al
Studio Brevetti e Marchi NOTARBARTOLO & GERVASI 33, Viale Bianca Maria
I-20122 Milano (IT)

EP 0 052 086 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a new process for producing flavone derivatives of formula:

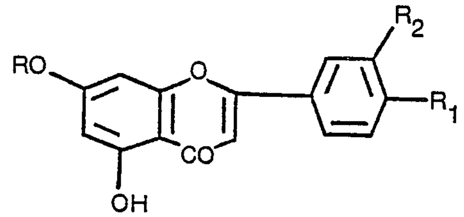

(I)

in which R is H or a disaccharide radical; $R_1$ is —OH or —$OCH_3$; $R_2$ is H or —OH.

In particular, the new process relates to the production of diosmin of formula:

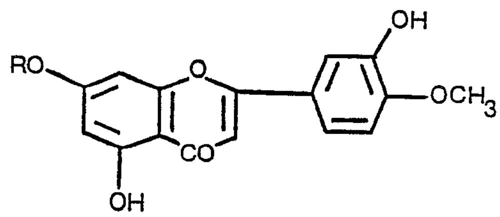

(II)

in which R is a rutinose residue (6-*O*-(6-deoxy-α-1-mannopyranosyl)-β-D-glucopyranosyl radical), and of apigenin of formula:

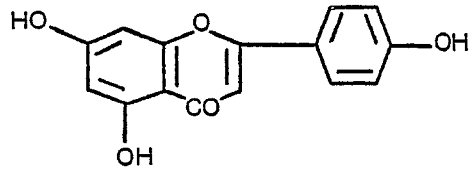

(III)

Both diosmin and apigenin are products which have been known for a very long time, as they are products of natural origin obtainable by extraction from various types of plant. Small quantities have been prepared by various processes generally used for the production of flavones, in order to determine their structure and test their activity.

In recent years both diosmin and apigenin have found various interesting pharmaceutical uses, which have posed the previously non-existent problem of producing said products industrially.

In particular, it has been found that diosmin is a product which is excellent for the venous system and as a hepato-protector, whereas apigenin has good spasmolytic activity both on the smooth musculature in general and on the bronchial musculature, in addition to having a good anti-inflammatory and anti-shock activity. Both these products have the great advantage of total lack of toxicity.

The problem of industrially producing diosmin and apigenin has proved difficult to solve, both with regard to the economics of the process and with regard to the purity of the final product.

In this respect, known processes generally use costly raw materials and always have very low yields. In addition, the products obtained contain small percentages of by-products related to the production process used, and are absolutely unacceptable in a pharmaceutical product.

A process has recently been proposed for diosmin which, in contrast to the previous processes, is considered an industrial process (DE—A—2 602 314 of 12.1.78). This process, which uses hesperidin as its starting substance, is characterised by the formation of the double bond in the 2-3 position by bromination and dehydrobromination.

A new industrial process has now been found, and forms the subject matter of the present invention, which enables compounds of formula (I), and in particular diosmin and apigenin, to be produced starting from low cost raw materials, with high yields and pharmaceutical purity.

The new process comprises the following essential stages:

1) total acetylation of flavanone-γ-glucoside of formula:

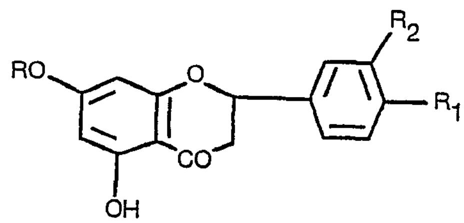

in which R is a disaccharide radical; $R_1$ is —OH or —$OCH_3$; $R_2$ is H or —OH, by heating to 90-100°C with acetic anhydride and pyridine;

2) oxidation of the acetyl derivative of the flavanone-7-glucoside prepared in the preceding stage, using selenium dioxide in isoamyl alcohol, in order to form a double bond in the 2—3 position by selective dehydrogenation, to give:

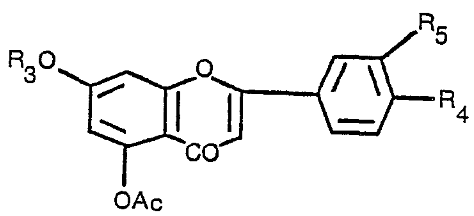

in which $R_3$ is a totally acetylated disaccharide radical; $R_4$ is —OAc or —$OCH_3$; $R_5$ is H or —OAc (Ac is an acetyl radical).

3) alkaline hydrolysis with dilute inorganic bases at 100°C in order to eliminate all the acetyl groups and restore the hydrosyl functions, to give:

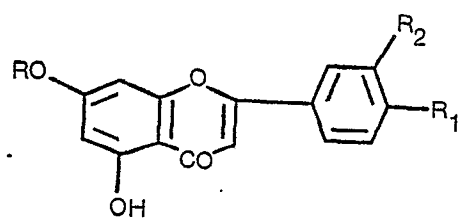

in which R, $R_1$, $R_2$ are as defined under point (1).

4) acid hydrolysis with dilute mineral acids under reflux, in order to separate the disaccharide radical R and form a hydroxy group in position 7, to give:

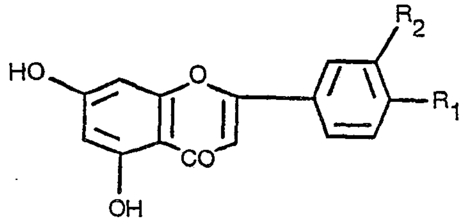

This stage is obviously optional, and is carried out only if the required product is an aglycone and not a glucoside.

If the process according to the present invention is applied to the production of diosmin, the starting substance is hesperidin or hesperetin-7-rhamnoglucoside of formula:

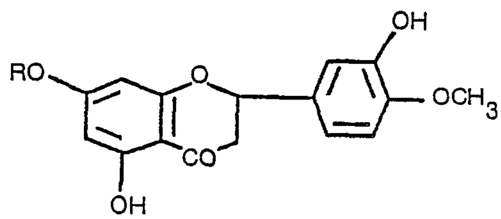

in which R is a rutinose radical (6-*O*-(6-deoxy-α-1-mannopyranosyl)-β-D-glucopyranosyl radical).

The octoacetyl derivative of hesperidin is prepared by heating with acetic anhydride and pyridine at 95°—100°C for 5—6 hours.

The hesperidin octoacetyl is precipitated by adding $H_2O$ to the reaction mixture and then cooling, and is purified by crystallisation. It is then dissolved in isoamyl alcohol and treated with an approximately 10

molar excess of $SeO_2$ under reflux for 24 hours.

The diosmin octoacetyl formed in this manner is hydrolysed with 15% NaOH by heating to 100°C for 10—15 minutes.

The diosmin of formula II precipitates on cooling and acidifying, and is purified by base-acid treatment.

If the process according to the present invention is applied to the production of apigenin, the starting substance used is naringin or naringenin-7-rhamnoglucoside of formula:

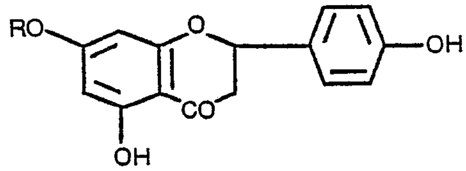

in which R is a rutinose radical (2-*O*-(6-deoxy-α-1-mannopyranosyl)-β-D-glucopyranosyl radical).

The naringin is totally acetylated by heating at 90—100°C for 5—6 hours with an excess of acetic anhydride and pyridine.

The octoacetyl naringin which precipitates by adding $H_2O$ to the reaction mixture and then cooling, is dissolved in isoamyl alcohol and treated with an approximately 10 molar excess of $SeO_2$ under reflux for 24 hours.

The unsaturated 2—3 octoacetyl derivative of formula

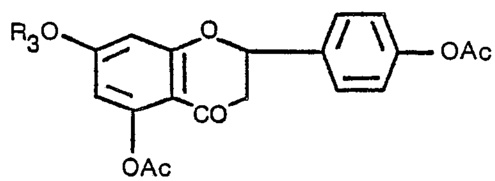

is formed, in which $R_3$ is a totally acetylated rutinose radical, and Ac is $—COCH_3$, and this is treated with dilute alkalis, preferably with 15% NaOH, at 100°C for 10—15 minutes.

The apigenin-7-rhamnoglucoside precipitates by cooling and acidifying with strong mineral acid, and is crystallised from methanol from which apigenin is obtained by hydrolysis with dilute mineral acids, preferably 18% $H_2SO_4$, under reflux.

Both the diosmin and apigenin obtained by the process according to the present invention are of pharmaceutical purity.

The final overall yield both of diosmin and apigenin is about 60%. A further important aspect of the process according to the present invention is the fact that the raw starting materials used are glucosides, in this case hesperidin and naringin, which are available in large quantity and low price in the discarded parts of citrus fruits. Compared with the only known industrial process for preparing diosmin (the said DE—A—2 602 314), the advantage of the new process is that the use of bromine is avoided, this being a reagent which at the industrial level creates great environmental hydiene and effluent pollution problems.

Two actual preparation examples are given hereinafter, for non-limiting illustration purposes only, in order to allow a more complete understanding and easy reproduction of the process according to the present invention.

Example 1

Synthesis of diosmin from hesperidin.

1) The following are fed into a 2 litre flask:

— 48 g of hesperidin (78.6 m moles)
— 600 ml of acetic anhydride
— 100 ml of pyridine.

The mixture is stirred and heated to 95—100°C for 5 hours. The flask contents are then poured into 6 litres flask, they are cooled with ice, and 4500 ml of iced water are slowly added.

The precipitate is filtered, washed with water, dried in an oven at 60—70°C, and crystallised from hot benzene and hexane. ·

55.82 g of white crystalline acetate (58.95 m moles) precipitate, having a M.P. of 123—125° (dec.).

Yield with respect to the theoretical 75%.

2) The following are fed into a 2 litre flask:

— 55.82 g of hesperidin octoacetyl (58.95 m moles)
— 65.5 g of $SeO_2$ (590 m moles)
— 1000 ml of isoamyl alcohol.

The mixture is heated under reflux for 24 hours, the metal Se which forms is then filtered off under hot conditions, and the solvent is evaporated.

3) 870 ml of 15% NaOH is added to the residue, and the mixture heated to 100°C for 10 minutes.

It is cooled, 150 ml of concentrated hydrochloric acid are slowly added, and the solution which is still alkaline is filtered.

The aqueous solution is extracted repeatedly with ethyl acetate, and then again acidified with concentrated HCl until a pH of 3 is attained.

A yellow solid precipitates, and this is purified by base-acid treatment and dried at 120°C.

28.4 g of diosmin are obtained having a M.P. of 278—280°C.

Yield with respect to the theoretical 79.1%.

On chromatographic analysis, the product was found to be totally free of impurities.

Example 2

Synthesis of apigenin from naringin.

1) The following are fed into a 2 litre flask:

— 48.25 g of naringin (83.11 m moles)

— 600ml of acetic anhydride

— 100 ml of pyridine.

The mixture is heated to 95—100°C for 5 hours, and is then poured into a 6 litre flask, cooled with ice and 4500 ml of iced water are added.

The precipitate is filtered off, washed several times with water, dried in an oven at 60—70°C and crystallised by dissolving in 400 ml of hot benzene, followed by crystallisation from hexane.

56 g of naringin octoacetyl are obtained, having a M.P. of 119—121°C (dec.).

Yield with respect to the theoretical 73.5%.

2) The following are fed into a 2 litre flask:

— 56 g of naringin octoacetyl (61.08 m moles)

— 77.8 g of $SeO_2$ (701.15 m moles)

— 1100 ml of isoamy alcohol

and the mixture heated under reflux for 24 hours.

The Se which forms is filtered off under hot conditions, and the solvent is evaporated.

870 ml of 15% NaOH are added to the residue, and the mixture is heated to 100°C for 10 minutes. It is cooled, 150 ml of concentrated hydrochloric acid are slowly added, and the solution which is still alkaline is filtered. The aqueous solution is extracted with 500 ml of ethyl acetate, and again with 300 ml of ethyl acetate. The aqueous solution is acidified with concentrated HCl until a pH of 3 is attained, and the solid precipitate is filtered off and dried at 100°C.

The apigenin-7-rhamnoglucoside is dissolved in boiling methanol, and the solution left at ambient temperature for 24—36 hours until the product has completely reprecipitated.

The solid precipitate is filtered off, is washed with iced methanol and is dried at 100°C.

28.25 g (48.83 m moles) of product are obtained, having a M.P. of 198°—200°C.

Yield with respect to the theoretical 80%.

3) The following are fed into a 3 litre flask:

— 28.25 of apigenin-7-rhamnoglucoside (48.83 m moles)

— 1600 ml of 18% sulphuric acid.

and the mixture heated under reflux for 4—5 hours.

On cooling, 13 g of a yellow crystalline product having a M.P. of 345°—350°C precipitate.

Yield with respect to the theoretical 98.52%.

On chromatographic analysis, the apigenin was found to be totally free from impurities of any kind.

**Claims**

1. A process for producing flavone derivatives of formula

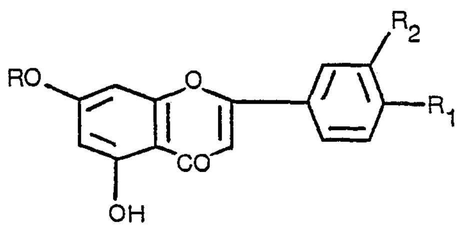

(I)

in which R is hydrogen or a disaccharide radical; $R_1$ is —OH or —$OCH_3$; $R_2$ is H or —OH. starting from flavanone-7-glucosides of formula:

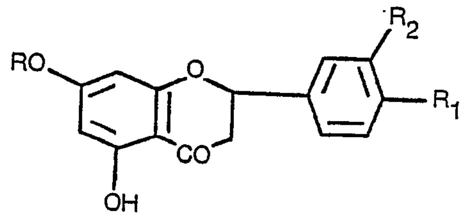

in which R, $R_1$, $R_2$ are as heretofore defined, by means of:

1) total acetylation of the flavanone-7-glucoside by heating to 90—100°C for about 5 hours with acetic anhydride and pyridine;

b) selective dehydrogenation in the 2—3 position by means of $SeO_2$ in an approximately 10 molar excess over the flavanone, by heating an isoamyl alcohol solution under reflux;

c) total deacetylation by means of alkaline hydrolysis with dilute inorganic bases at 100°C;

d) optional elemination of the glucoside radical by means of acid hydrolysis with dilute mineral acids under reflux.

2. A process as claimed in claim 1, wherein diosmin is prepared from hesperidin.

3. A process as claiemd in claim 1, wherein apigenin is prepared from naringin.

4. A process as claimed in claim 1, wherein the deacetylation by alkaline hydrolysis is carried out with 15% NaOH at 100°C for 10 minutes.

5. A process as claimed in claim 1, wherein the elimination of the glucoside radical by acid hydrolysis is carried out with 18% dilute sulphuric acid under reflux for 4—5 hours.

**Revendications**

1. Procédé pour la préparation de dérivés de la flavone de formule

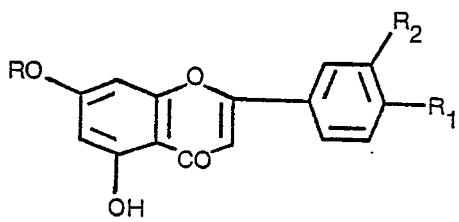

(I)

dans laquelle R est un atome d'hydrogène ou un radical disaccharide; $R_1$ est mis pour —OH ou —$OCH_3$; $R_2$ est mis pour H ou —OH, à partir de flavanone-7-glucosides de formule

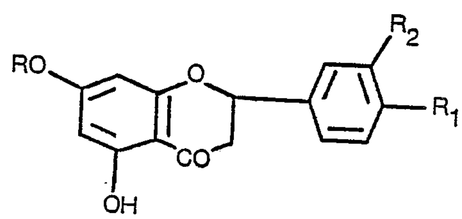

(a)

dans laquelle R, $R_1$, $R_2$ ont les significations données ci-dessus, par le moyen de:

a) acétylation totale du flavanone-7-glucoside par chauffage à 90—100°C pendant 5 h environ avec l'anhydride acétique et la pyridine;

b) déshydrogénation sélective en position 2—3, au moyen de $SeO_2$ utilisé en un excés approximativement 10 molaire par rapport à la flavanone, par chauffage d'une solution dans l'alcool isoamylique au reflux;

c) désacétylation totale par hydrolyse alcaline avec une base minérale diluée à 100°C;

d) le cas échéant, élimination du radical glucoside par hydrolyse acide avec un acide minéral diluée au reflux.

2. Procédé selon la revendication 1, dans lequel la diosmine est préparée à partir d'hespéridine.

3. Procédé selon la revendication 1, dans lequel l'apigénine est préparée à partir de naringine.

4. Procédé selon la revendication 1, dans lequel la désacétylation par hydrolyse alcaline est effectuée avec NaOH à 15%, à 100°C pendant 10 minutes.

5. Procédé selon la revendication 1, dans lequel l'élimination du radical glucoside par hydrolyse acide est effectuée avec l'acide sulfurique dilué à 18%, au reflux pendant 4 à 5 heures.

**Patentansprüche**

1. Verfahren zum Herstellen von Flavonderivaten der Formel

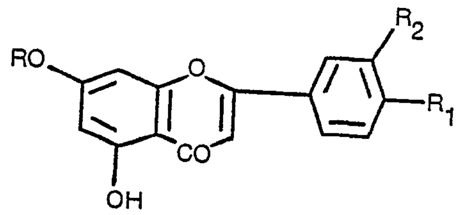

(I)

worin R Wasserstoff oder ein Disaccharidrest ist; $R_1$ —OH oder —$OCH_3$ bedeutet und $R_2$ H oder —OH darstellt, ausgehend von Flavanon-7-glukosiden der Formel

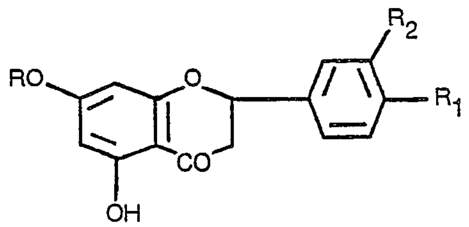

worin R, $R_1$ und $R_2$ wie oben definiert sind, mittels

a) Gesamtacetylierung des Flavanon-7-glukosids durch Erhitzen während etwa 5 h auf 90 bis 100°C mit Acetanhydrid und Pyridin,

b) selektive Dehydrierung in der 2—3-Stellung mittels $SeO_2$ in einem annähernd 10 molaren Überschuß gegenüber dem Flavanon durch Erhitzen einer Isoamylalkohollösung unter Rückfluß,

c) Gesamtdeacetylierung mittels alkalischer Hydrolyse mit verdünnten anorganischen Basen bei 100°C, und

d) gegebenenfalls Entfernung des Glukosidrestes mittels saurer Hydrolyse mit verdünnten Mineralsäuren am Rückfluß.

2. Verfahren, wie in Anspruch 1 beansprucht, worin Diosmin aus Hesperidin hergestellt wird.

3. Verfahren, wie in Anspruch 1 beansprucht, worin Apigenin aus Naringin hergestellt wird.

4. Verfahren, wie in Anspruch 1 beansprucht, worin die Deacetylierung mittels alkalischer Hydrolyse mit 15 %iger NaOH bei 100°C während 10 min durchgeführt wird.

5. Verfahren, wie in Anspruch 1 beansprucht, worin die Entfernung des Glukosidrestes durch saure Hydrolyse mit 18 %iger verdünnter Schwefelsäure am Rückfluß während 4 bis 5 h durchgeführt wird.